(19) Europäisches Patentamt

European Patent Office

Office européen des brevets

(11) Veröffentlichungsnummer: **0 491 969 B1**

(12) **EUROPÄISCHE PATENTSCHRIFT**

(45) Veröffentlichungstag der Patentschrift: **23.08.95**

(51) Int. Cl.⁶: **A61M 16/00**

(21) Anmeldenummer: **90124930.0**

(22) Anmeldetag: **20.12.90**

(54) **Beatmungsgerät mit vom Patientengasfluss abhängiger Triggerempfindlichkeit.**

(43) Veröffentlichungstag der Anmeldung:
**01.07.92 Patentblatt  92/27**

(45) Bekanntmachung des Hinweises auf die
Patenterteilung:
**23.08.95 Patentblatt  95/34**

(84) Benannte Vertragsstaaten:
**CH DE ES FR GB IT LI NL SE**

(56) Entgegenhaltungen:
**EP-A- 0 046 570**
**EP-A- 0 402 951**
**FR-A- 2 596 279**

**Puritan Bennetts Option 50 (20535A 10-86)**

(73) Patentinhaber: **Siemens Elema AB**
**Röntgenvägen 2**
**S-171 95 Solna 1 (SE)**

(84) Benannte Vertragsstaaten:
**SE**

(73) Patentinhaber: **SIEMENS AKTIENGESELL-**
**SCHAFT**
**Wittelsbacherplatz 2**
**D-80333 München (DE)**

(84) Benannte Vertragsstaaten:
**CH DE ES FR GB IT LI NL**

(72) Erfinder: **Olsson, Sven Gunnar, Dipl.-Ing.**
**Villa Fortuna**
**S-232 00 Arlöv (SE)**
Erfinder: **Rydgren, Göran, Dipl.-Ing.**
**Strandängsvägen 4**
**S-230 44 Bunkeflostrand (SE)**
Erfinder: **Lindén, Dan, Dipl.-Ing.**
**Dianavägen 35**
**S-115 43 Stockholm (SE)**

Rank Xerox (UK) Business Services
(3.10/3.09/3.3.3)

## Beschreibung

Die Erfindung betrifft ein Beatmungsgerät gemäß dem Oberbegriff des ersten Anspruches. Ein Beatmungsgerät mit einstellbarer Triggerempfindlichkeit für den Druck in der Exspirationsleitung ist beispielsweise aus der Gebrauchsanweisung für den Servoventilator 900 D, zweite deutsche Ausgabe, Oktober 1988 bekannt. Das Beatmungsgerät mißt in der Exspirationsleitung den Druck. Wenn dieser die eingestellte Triggerem-pfindlichkeit dadurch unterschreitet, daß beispielsweise ein Patient durch eine versuchte Einatmung den entsprechenden Unterdruck erzeugt, schaltet das Beatmungsgerät automatisch von der Exspirationsphase auf die Inspirationsphase um. In vielen Fällen wird während der Exspirationsphase mit einem positiven endexspiratorischem Druck, PEEP gearbeitet, um zum Beispiel dem Auftreten von Atelektasen vorzubeugen. Der PEEP- Einstellbereich kann z.B. von 0 bis 50 mbar reichen. Die Triggerempfindlichkeit ist damit vom PEEP- Niveau abhängig. Wird beispielsweise ein PEEP- Niveau von +10 mbar gewählt und eine Triggerempfindlichkeit von -2 mbar, so bedeutet das, daß ein Patient im Vergleich zur Einstellung des PEEP - Niveaus einen Unterdruck von 2 mbar erzeugen muß, um einen Atemzug zu triggern.

Die Triggerempfindlichkeit kann mit Hilfe eines Drehknopfes auf einen festen Wert eingestellt werden. Diese Einstellung kann unter Umständen schwierig sein. Wird die Triggerempfindlichkeit zu klein gestellt, daß heißt der Abstand zum PEEP - Niveau zu gering gewählt, so können bereits Druckschwankungen in der Exspirationsleitung zur Auslösung eines neuen Atemzuges ausreichen. Das sollte natürlich vermieden werden. Andererseits kann eine zu große Triggerempfindlichkeit, daß heißt ein zu großer Abstand des Triggerniveaus vom PEEP - Niveau dazu führen, daß der Patient bei versuchter Einatmung eine erhebliche Kraft aufwenden muß, um den nötigen Unterdruck in der Exspirationsleitung zu erzeugen. Auch das ist natürlich unerwünscht. Insbesondere durch den Gasfluß von der Inspirationsleitung auch während der Exspirationsphase, den sogenannten Bypass Gasfluß, kann der Patient zunächst einmal diese Gasmenge einatmen, ohne daß es zu einen Druckabfall in der Exspirationsleitung kommt.

Die Anwendung des Bypass Gasflusses ist beispielsweise aus der US-A-4 401 116 bekannt. Ebenso wird das Betreiben eines Beatmungsgerätes mit Bypass Gasfluß in der gattungsgemäßen Druckschrift Puritan Bennetts Option 50 (20535A 10-86) beschrieben. Diese Option ist als "Flow-by" bezeichnet. Im Gegensatz zur eingangs beschriebenen druckabhängigen Triggerung eines neuen Atemzuges wird hier eine gasflußabhängige Triggerung beschrieben. Mit Hilfe von zwei Gasflußsensoren wird der Gasfluß in der Inspirations- und in der Exspirationsleitung gemessen und daraus der Nettogasfluß bestimmt. Dieser entspricht dem Patiengasfluß, das heißt dem Gasfluß, der entweder bei der Einatmung vom Patienten aufgenommen wird oder bei der Ausatmung vom Patienten abgegeben wird. Während der Atempause, daß heißt wenn der Patient weder ein- noch ausatmet, ist der Nettogasfluß Null.

Aus der EP-A-0 402 951 ist ein Beatmungsgerät bekannt. Das Beatmungsgerät kontrolliert die Zufuhr eines Atmungsgases zu einem Patenten aufgrund eines gemessenen respiratorischen Gasflusses des Patienten. Der Gasfluß kann mit einem oder zwei Flußmessern gemessen werden. Das Beatmungsgerät kann mit druckregelnden Mitteln ausgeführt sein. Der Luftwegdruck des Patienten kann hier aufgrund des gemessenen Gasflusses geregelt sein.

Wenn der Patient anfängt einzuatmen, gelangt ein Teil des Bypass Gasflußes in die Lungen des Patienten und der Exhalationsgasfluß wird entsprechend reduziert. Der Nettogasfluß oder der Patientengasfluß wird damit negativ. Wenn dieser Wert einen einstellbaren Empfindlichkeitswert erreicht, wird ein neuer Atemzug ausgelöst. Aus Sicherheitsgründen ist daneben immer noch die druckabhängige Triggerung vorgesehen, sozusagen als Back-up. Diese beiden parallelgeschalteten Triggermethoden machen die Einstellung und die Handhabung noch komplizierter.

Der vorliegenden Erfindung liegt die Aufgabe zugrunde, bei einem Beatmungsgerät der eingangs genannten Art das Auslösen eines neuen Atemzuges während der Exspirationsphase zu verbessern. Dieser Aufgabe wird erfindungsgemäß durch die Merkmale des Anspruchs 1 gelöst.

Im Gegensatz zu den bisherigen Methoden, entweder druckempfindlich zu Triggern oder in Abhängigkeit von dem Nettogasfluß durch das Beatmungsgerät, wird hier eine geschickte Kombination beider Triggermethoden vorgeschlagen, die die Triggerung zuverläßiger und leichter anwendbar macht. Die Kombination geht dabei darauf hinaus, daß gewissermaßen bei der druckabhängigen Triggerung die Triggerempfindlichkeit über den Patientengasfluß, oder anders ausgedrückt den Nettogasfluß durch das Beatmungsgerät, verändert wird. Vorteilhafterweise wird dabei die Triggerempfindlichkeit kleiner, daß heißt der notwendige Unterdruck zur Auslösung eines neuen Atemzuges geringer, wenn der Nettogasfluß negativ wird.

In einer vorteilhaften Weiterbildung der Erfindung ist dazu vorgesehen, daß sowohl in der Inspiration- als auch in der Exspirationsleitung ein Flußmesser vorgesehen ist, sowie Mittel, die daraus den Nettogasfluß ermitteln. Andererseits ist es im Rahmen der Erfindung auch möglich, einen einzigen Flußmesser dicht an den Atemwegen oder zumindest in einer von Exspirations- und Inspirationsleitung gemeinsamen Verbindungsleitung zum Patienten anzuordnen, der sowohl die Größe als auch die Richtung der Fluß es durch diese Leitung angibt.

In Weiterbildung der Erfindung ist vorgesehen, daß die maximale Änderung des Triggerniveaus, die dann erreicht wird, wenn der gesamte Bypas - Gasfluß vom Patienten eingeatmet und der Exspirationsfluß damit Null wird, größer ist als das eingestellte Triggerniveau für den Nettogasfluß Null. Für diesen Fall wird aus der druckabhängigen Triggerung quasi eine gasflußabhängige Triggerung, wenn man voraussetzt, daß der Druck korrekt ist, das heißt, daß (PEEP -$P_{EXSP}$) = 0 ist. Die vorab eingestellte druckabhängige Triggerempfindlichkeit bestimmt nun, bei welchem Patientengasfluß oder Nettogasfluß die Auslösung eines neuen Atemzuges geschieht. Abhängig von der Stärke der Einatmung durch den Patienten verschiebt sich die Triggerempfindlichkeit gegen das PEEP - Niveau und die Auslösung erfolgt, wenn Triggerniveau und PEEP - Niveau oder besser ausgedrückt wenn Triggerniveau und der Druck in der Exspirationsleitung gleich sind. Der Vorteil besteht dabei darin, daß der Patient durch die Einatmung des Bypass Gasfluß es bereits die Triggerung auslösen kann und daß es nicht notwendig ist, daß die Einatmungsanstrengungen dabei so groß sind, daß ein Unterdruck in der Exspirationsleitung erzeugt wird. Auf der anderen Seite wird während der Ausatmung durch den Patienten das Triggerniveau und damit die Empfindlichkeit gesenkt, so daß Druckschwankungen insbesondere am Anfang der Exspirationsphase keine falschen Triggerungen hervorrufen können.

Wenn die Triggerempfindlichkeit zu Anfangs großer eingestellt wird als die mögliche positive Veränderung derselben, liegt stets nur eine druckabhängige Triggerung vor, jedoch auch hier mit durch den Fluß veränderlicher Empfindlichkeit.

Weitere Vorteile und Ausgestaltungen ergeben sich aus den Unteransprüchen sowie aus dem nachfolgenden Ausführungsbeispiel, in dem die Erfindung näher beschrieben und erläutert ist. Dabei zeigen
Fig. 1 ein erfindungsgemäßes Beatmungsgerät und
Fig. 2 die Abhängigkeit des Triggerniveaus von unterschiedlichen Patientengasflüssen.

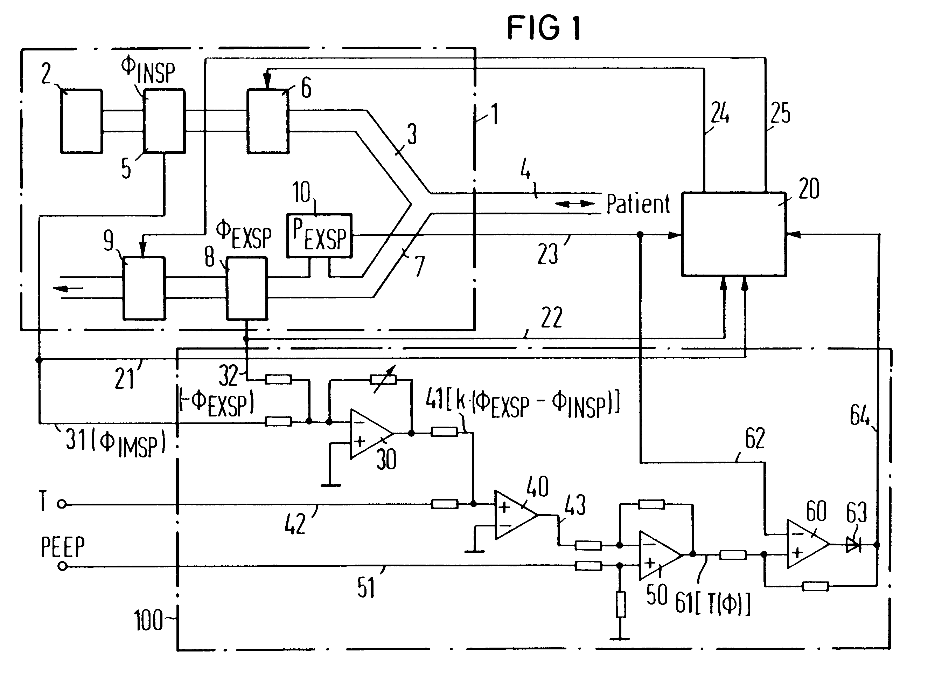

Im oberen Teil der Fig. 1 ist gestrichelt ein Ventilator 1 dargestellt, wie er beispielsweise aus der eingangs genannten Schrift von Puritan Bennett, Option 10, bekannt ist. Schematisch sind nur die für die Erfindung wesentlichen Teile angegeben.

Von einer Gasversorgung 2 wird das Gas über eine Inspirationsleitung 3 einer zu einem Patienten führenden Leitung 4 zugeführt. In der Inspirationsleitung 3 ist ein Flußmesser 5 und ein Regelventil 6 für den Gasfluß, beispielsweise ein Solenoidventil angeordnet.

Mit der gemeinsamen Leitung 4 ist eine Exspirationsleitung 7 verbunden, an die ein weiterer Flußmesser 8, ein Regelventil 9 und ein Druckmesser 10 angeschlossen sind.

Weiterhin ist in Fig 1 eine Steuervorrichtung 20 als Block dargestellt, mit der in bekannter Weise sämtliche Funktionen des Beatmungsgerätes geregelt werden und an der die gewünschten Grenzparameter eingestellt werden können. Dazu sind zumindest die von den Flußmessern 5 und 8 und dem Druckmesser 10 ermittelten Werte als elektrische Signale über Leitungen 21, 22 und 23 auf die Steuervorrichtung 20 geschaltet Über weitere Leitungen 24 und 25 werden die Regelventile 6 beziehungsweise 9 angesteuert. Die Steuervorrichtung kann analog aufgebaut sein. Ebenso ist es möglich, die Eingangssignale zu digitalisieren und die Bearbeitung mit Hilfe eines Mikroprozessors vorzunehmen. In dem Ausführungsbeispiel der Fig. 1 ist eine analoge Triggervorrichtung 100 zur Auslösung eines neuen Atemzuges angegeben. Auch hier sei erwähnt, daß es im Rahmen der Erfindung ebenso möglich ist, die einzelnen Meßwerte und Parameter wie Triggerempfindlichkeit oder PEEP zu digitalisieren und die Triggerung durch einen Mikroprozessor ermitteln zu lassen.

Über Leitungen 31 beziehungsweise 32 werden die Ausgangssignale der Flußmesser 5 beziehungsweise 8 auf einen ersten Verstärker 30 mit einstellbarem Verstärkungsfaktor gegeben; über die Leitung 31 der Wert für $\Phi_{INSP}$ und über die Leitung 32 der Wert für -$\Phi_{EXSP}$ gegeben ist. Auf der Ausgangsleitung 41 des Verstärkers 30 liegt somit ein Signal entsprechend der Größe k x $\Phi_{EXSP}$ -$\Phi_{INSP}$ vor. Dabei ist k der einstellbare Verstärkungsfaktor. Im Verstärker 40 wird zu diesem Wert das eingestellte Triggerniveau T addiert, das über eine Leitung 42 auf den Verstärker 40 geschaltet ist. In dem nachfolgenden Differenzverstärker 50 wird die Differenz zwischen einem dem PEEP entsprechenden Signal und dem am Ausgang des Verstärkers 40 auf der Leitung 43 vorliegenden Signal gebildet. Das PEEP entsprechende Signal wird über eine Leitung 51 auf den positiven Eingang des Differenzverstärkers 50, das Ausgangssignal des Verstärkers 40 auf den negativen Eingang des Differenzverstärkers 50 geschaltet. Auf der Ausgangsleitung 61 des Differenzverstärkers 50 liegt damit ein dem vom Gasfluß abhängigen Triggerniveau entsprechendes Signal vor. Dieses wird in einem Differenzverstärker 60 mit dem über eine Leitung 62 auf diesen Differenzverstärker geschalteten Signal des Druckmessers 10 in der Exspirationsleitung verglichen. Wenn der Exspirationsdruck gleich dem variablen Triggerniveau ist, wird über eine Diode 63 und eine Leitung 64 ein Triggersignal auf die Steuervorrichtung 20 gegeben, die dadurch das Exspirationsventil schließt und das Inspirationsventil entsprechend der eingestellten Beatmungsform öffnet und damit einen neuen Atemzug einleitet.

Die in den Leitungen angeordneten Widerstände dienen lediglich der Einstellung der richtigen Verstärkung und der richtigen Arbeitspunkte und sind daher nicht näher erläutert.

Diese Auswertschaltung ermittelt damit folgenden Wert:

$$PEEP + T + k \times (\Phi_{EXSP} - \Phi_{INSP}) - P_{EXSP}.$$

Wenn dieser Ausdruck kleiner 0 wird, wird ein neuer Atemzug getriggert. Der Verstärkungsfaktor k ist dabei so gewählt, daß der Ausdruck $k \times (\Phi_{EXSP} - \Phi_{INSP})$ für den Fall, daß das gesamte Bypass - Gas vom Patienten eingeatmet wird, einer Erhöhung des variablen Triggerniveaus T ($\Phi$) um einem bestimmten Betrag entspricht. Dieser Betrag kan so groß gewählt werden, daß er größer ist als der Abstand zwischen PEEP und T für $\Phi_{EXSP} = \Phi_{INSP}$.

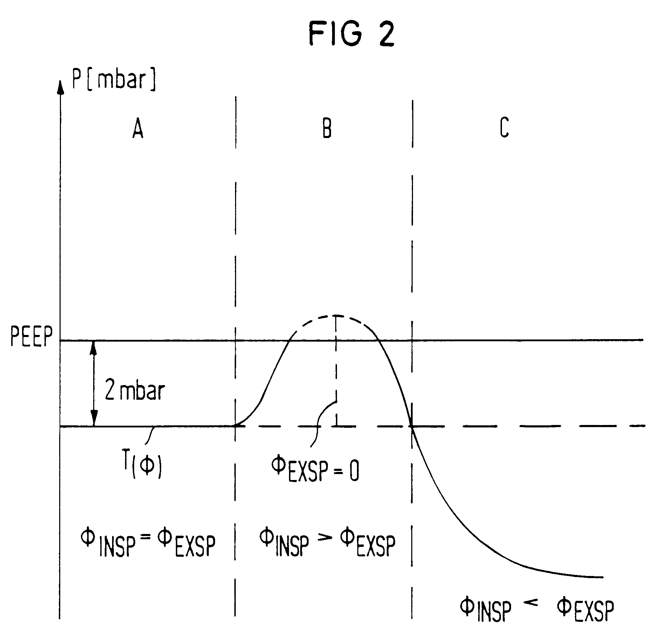

In Fig. 2 sind die Druckverhältnisse über der Zeit für verschiedene Gasflüsse angegeben. Ausgegangen wird dabei vom PEEP, der als horizontale Zeitachse dargestellt ist. Dieser kann 0 mbar betragen oder auf einen einstellbaren Wert zwischen 0 und einigen mbar festgelegt sein. Die Triggerempfindlichkeit T ($\Phi$) ist vom PEEP - Niveau abhängig. Sie ist jeweils relativ zum PEEP - Niveau angegeben. Eine Triggempfindlichkeit von -2 mbar beispielsweise entspricht einer Absenkung dieses Triggerniveaus um 2 mbar unter das PEEP - Niveau, wie es beispielsweise im linken Drittel der Fig. 2 angedeutet ist. Das Triggerniveau ist für diesen Bereich A als konstant angesetzt. Eine Beeinflussung durch den Fluß liegt hier nicht vor, da angenommen ist, daß der Inspirationsfluß gleich dem Exspirationsfluß ist und damit der Nettogasfluß 0 wird. Dieses konstante Triggerniveau ist gestrichelt auch über die Bereiche B und C fortgesetzt. Dieses Niveau würde bei der bekannten reinen druckabhängigen Triggerung gelten. Das Triggerniveau würde dabei von Hand auf einen bestimmten Wert eingestellt.

Im mittleren Bereich B der Fig. 2 ist das Triggerniveau für den Fall angegeben, in dem ein Nettogasfluß zum Patienten vorliegt, das heißt, daß der Patient spontan eine Einatmung beginnt. Wie man dieser Figur entnehmen kann, nähert sich dadurch das Triggerniveau je nach der Größe dieser Einatmung dem PEEP - Niveau und kann es theoretisch, wie gestrichelt angedeutet, sogar überschreiten. Der Maximalwert würde vorliegen, wenn der Exspirationsfluß Null ist, das heißt, wenn das gesamte Bypass - Gas vom Patienten eingeatment wird. Da bei korrekten Druckverhältnissen zumindest am Ende der Exspirationsphase der Druck in der Exspirationsleitung dem PEEP entspricht, wird ein neuer Atemzug getriggert, wenn das Triggerniveau T ($\Phi$) das PEEP - Niveau erreicht. In diesem Bereich liegt also eine gasflußabhängige Triggerung vor. Es ist nicht notwendig, daß der Patient einen Unterdruck gegenüber dem PEEP erzeugt. Die spontante Auslösung eines neuen Atemzuges wird damit für den Patienten erheblich erleichtert und sie wird wesentlich früher eingeleitet als es mit einer reinen druckabhängigen Triggerung möglich wäre. Bei dieser müßte der Patient zunächst das gesamte Bypass - Gas einatmen und eine Anstrengung unternehmen noch mehr Gas einzuatmen, bevor der Druck in der Exspirationsleitung unter das PEEP - Niveau und bis zu dem gegen dieses verschobene Triggerniveau absinken würde.

Im Abschnitt C dieser Fig. 2 ist das Triggerniveau für den Fall angegeben, daß der Inspirationsfluß kleiner als der Exspirationsfluß ist, das heißt, daß der Patient ausatmet. Das Triggerniveau wird dadurch weiter von dem PEEP - Niveau entfernt. Die Triggerempfindlichkeit wird dadurch verringert. Dadurch wird sicher verhindert, daß Druckschwankungen zu Beginn der Ausatmungsphase ungewollt einen neuen Atemzug triggern können.

Die einstellbare und dem Nettogasfluß Null entsprechende Triggerempfindlichkeit kann so vergrößert werden, daß selbst bei maximaler Einatmung des gesamten Bypass - Gasflusses die Erhöhung des Triggerniveaus nicht ausreicht, um das PEEP - Niveau zu erreichen. In diesem Fall ist dann zwar keine gasflußempfindliche Triggerung mehr möglich, weiterhin wird aber das Triggerniveau für den notwendigen Unterdruck angehoben, so daß die druckabhängige Triggerung wesentlich empfindlicher wird.

**Patentansprüche**

1. Beatmungsgerät mit einer Inspirations- und einer Expirationsleitung (3,7) zum Anschließen an die Atemwege von Menschen oder Tieren, einem in der Inspirationsleitung (3) vorgesehenen ersten Ventil (6), mit dessen Hilfe der Gasfluß durch die Inspirationsleitung einstellbar ist, einem in der Exspirationsleitung (7) vorgesehenen zweiten Ventil (9), mit dem der Gasfluß durch die Exspirationsleitung einstellbar ist, einer Steuervorrichtung (20) zur Betätigung der beiden Ventile derart, daß ein Atemzyklus mit Inspirations- und Exspirationsphase erzeugbar ist und wobei während der Exspirationsphase das erste Ventil (6) soweit geöffnet bleibt, daß ein einstellbarer Bypass - Gasfluß erzeugt wird, sowie mindestens einem Flußmesser (5,8) zum Bestimmen des Patientengasflusses, mindestens einem Druckmesser (10) zur Bestimmung des Exspirationsdruckes und einer Triggervorrichtung (100), die während der Exspirationsphase beim Unterschreiten des Druckes in der Exspirationsleitung unter einen vorgegebenen Wert eine neue Inspirationsphase auslöst, **dadurch gekennzeichnet**, daß der der Triggervorrichtung (100) vorgegebene Wert des Druckes in der Exspirationsleitung in Abhängigkeit von dem durch den Flußmesser (5,8) bestimmten Patientengasfluß variierbar ist.

2. Beatmungsgerät nach Anspruch 1 **dadurch gekennzeichnet**, daß der der Triggervorrichtung (100) vorgegebene Wert so variierbar ist, daß er kleiner wird, wenn der Patientengasfluß vom Patienten weggerichtet ist und umgekehrt.

3. Beatmungsgerät nach Anspruch 1 oder 2, **dadurch gekennzeichnet**, daß in bekannter Weise in der Inspirations- und der Exspirationsleitung (3,7) jeweils ein Flußmesser (5,8) angeordnet ist und das der Patientengasfluß als Differenz zwischen dem Exspirationsgasfluß $\Phi_{EXSP}$ und dem Inspirationsgasfluß $\Phi_{INSP}$ gebildet wird.

4. Beatmungsgerät nach einem der Ansprüche 1 bis 3, **dadurch gekennzeichnet**, daß der vor-gegebene Wert die Triggerempfindlichkeit be-rücksichtigt.

5. Beatmungsgerät nach einem der Ansprüche 1 bis 4, **dadurch gekennzeichnet**, daß die maximale positive Änderung des vorgegebenen Wertes größer oder gleich dem Betrag der Triggerempfindlichkeit für den Fall ist, daß $\Phi_{EXSP} = \Phi_{INSP}$.

6. Beatmungsgerät nach einem der Ansprüche 1 bis 5, **dadurch gekennzeichnet**, daß der bzw. die Flußmesser (5,8) in Abhängigkeit von dem gemessenen Fluß ein elektrisches Signal erzeugen, daß der Druckmesser (10) ein dem vorliegenden Druck in der Exspirationsleitung entsprechendes elektrisches Signal erzeugt, und daß der vorgegebene Wert ebenfalls als elektrisches Signal vorliegt.

7. Beatmungsgerät nach Anspruch 6, **dadurch gekennzeichnet**, daß sämtliche elektrische Signale elektrische Spannungen sind.

8. Beatmungsgerät nach Anspruch 6 oder 7, **dadurch gekennzeichnet**, daß die den Gasflüssen entsprechenden elektrischen Signale auf einen ersten Verstärker (30) geschaltet sind, dessen Ausgangssignal zusammen mit dem elektrischen Signal für die Triggerempfindlichkeit auf einen weiteren Verstärker (40) geschaltet sind, dessen Ausgangssignal auf einen Eingang eines ersten Differenzverstärkers (60) ge-schaltet ist, an dessen anderen Eingang das dem Druck in der Exspirationsleitung entsprechende elektrische Signal anliegt.

9. Beatmungsgerät nach Anspruch 8, **dadurch gekennzeichnet**, daß zwischen dem weiteren Verstärker (40) und dem ersten Differenzverstärker (60) ein weiterer Differenzverstärker (50) geschaltet ist, an dessen anderem Eingang das dem PEEP entsprechende elektrische Signal anliegt.

10. Beatmungsgerät nach Anspruch 8 oder 9, **da-durch gekennzeichnet**, daß der Verstär-kungsfaktor k des ersten Verstärkers (30) einstellbar ist.

**Claims**

1. Ventilator having an inspiration and an expiration line (3, 7) for connecting to the airways of humans or animals, a first valve (6), provided in the inspiration line (3), with the aid of which the gas flow through the inspiration line can be adjusted, a second valve (9), provided in the expiration line (7), with the aid of which the gas flow through the expiration line can be adjusted, a control device (20) for operating the two valves in such a way that a respiration cycle with inspiration and expiration phase can be produced, it being the case that during the expiration phase the first valve (6) remains open to such an extent than an adjustable bypass gas flow is generated, as well as at least one flow meter (5, 8) for determining the gas flow of the patient, at least one pressure gauge (10) for determining the expiration pressure and a trigger device (100) which during the expiration phase triggers a new inspiration phase upon undershooting of the pressure in the expiration line below a prescribed value, characterized in that the value of the pressure in the expiration line, which value is prescribed for the trigger device (100), can be varied as a function of the gas flow of the patient determined by the flow meter (5, 8).

2. Ventilator according to Claim 1, characterized in that the value prescribed for the trigger device (100) can be varied in such a way that it becomes smaller when the gas flow of the patient is directed away from the patient, and vice versa.

3. Ventilator according to Claim 1 or 2, characterized in that in a known way a flow meter (5, 8) is respectively arranged in the inspiration and expiration lines (3, 7) and in that the gas flow of the patient is formed as the difference between the expiration gas flow $\Phi_{EXSP}$ and the inspiration gas flow $\Phi_{INSP}$.

4. Ventilator according to one of Claims 1 to 3, characterized in that the prescribed value takes account of the trigger sensitivity.

5. Ventilator according to one of Claims 1 to 4, characterized in that the maximum positive change in the prescribed value is greater than or equal to the absolute value of the trigger sensitivity for the case in which $\Phi_{EXSP} = \Phi_{INSP}$.

6. Ventilator according to one of Claims 1 to 5, characterized in that the flowmeter(s) (5, 8) generate an electric signal as a function of the measured flow, in that the pressure gauge (10) generates an electric signal corresponding to the pressure present in the expiration line, and in that the prescribed value is likewise present as an electric signal.

7. Ventilator according to Claim 6, characterized in that all the electric signals are electric voltages.

8. Ventilator according to Claim 6 or 7, characterized in that the electric signals corresponding to the gas flows are connected to a first amplifier (30) whose output signal is connected together with the electric signal for the trigger sensitivity to a further amplifier (40) whose output signal is connected to an input of a first differential amplifier (60) at whose other input the electric signal corresponding to the pressure in the expiration line is present.

9. Ventilator according to Claim 8, characterized in that there is connected between the further amplifier (40) and the first differential amplifier (60) a further differential amplifier (50) at whose other input the electric signal corresponding to the PEEP is present.

10. Ventilator according to Claim 8 or 9, characterized in that the gain k of the first amplifier (30) can be adjusted.

**Revendications**

1. Appareil respiratoire comportant un conduit d'inspiration et un conduit d'expiration (3,7) destinés à être raccordés aux voies respiratoires d'hommes ou d'animaux, une première soupape (6) prévue dans la conduite d'inspiration (3) et à l'aide de laquelle le flux gazeux dans la conduite d'inspiration peut être réglé, une seconde soupape (9) prévue dans la conduite d'expiration (7) et au moyen de laquelle le flux gazeux dans la conduite d'expiration peut être réglé, un dispositif de commande (20) pour actionner les deux soupapes de telle sorte qu'un cycle respiratoire puisse être produit avec des phases d'inspiration et d'expiration, la première soupape (6) restant ouverte pendant la phase d'expiration jusqu'à ce qu'un flux gazeux de dérivation réglable soit produit, ainsi qu'au moins un appareil de mesure du flux (5,6) servant à déterminer le flux gazeux du patient, au moins un manomètre (10) pour déterminer la pression d'expiration et un dispositif de déclenchement (100), qui, pendant la phase d'expiration, déclenche une nouvelle phase d'inspiration, lorsque la pression dans la conduite d'expiration tombe au-dessous d'une valeur prédéterminée, caractérisé par le fait que la valeur de la pression, qui est prédéterminée par le dispositif de déclenchement (100) et est présente dans la conduite d'expiration, peut être modifiée en fonction du flux gazeux du patient, déterminé par l'appareil de mesure du flux (5,8).

2. Appareil respiratoire suivant la revendication 1, caractérisé par le fait que la valeur prédéterminée pour le dispositif de déclenchement (100) peut être modifiée de manière à diminuer lorsque le flux gazeux du patient est dirigé en direction s'éloignant du patient, et inversement.

3. Appareil respiratoire suivant la revendication 1 ou 2, caractérisé par le fait que, de façon connue, un appareil de mesure du flux (5,8) est disposé respectivement dans la conduite d'inspiration et dans la conduite d'expiration (3,7) et que le flux gazeux du patient est formé en tant que différence entre le flux gazeux d'expiration $\Phi_{EXSP}$ et le flux gazeux d'inspiration $\Phi_{INSP}$.

4. Appareil respiratoire suivant l'une des revendications 1 à 3, caractérisé par le fait que la valeur prédéterminée prend en compte la sensibilité de déclenchement.

**5.** Appareil respiratoire suivant l'une des revendications 1 à 4, caractérisé par le fait que la variation maximale positive de la valeur prédéterminée est supérieure ou égale à la valeur absolue de la sensibilité de déclenchement dans le cas où l'on a $\Phi_{EXSP} = \Phi_{INSP}$.

**6.** Appareil respiratoire suivant l'une des revendications 1 à 5, caractérisé par le fait que le ou les appareils de mesure du flux (7,8) produisent un signal électrique en fonction du flux mesuré, que le débitmètre (10) produit un signal électrique qui correspond à la pression présente dans la conduite d'expiration et que la valeur prédéterminée est présente également sous la forme d'un signal électrique.

**7.** Appareil respiratoire suivant la revendication 6, caractérisé par le fait que tous les signaux électriques sont des tensions électriques.

**8.** Appareil respiratoire suivant la revendication 6 ou 7, caractérisé par le fait que les signaux électriques, qui correspondent aux flux gazeux, sont appliqués à un premier amplificateur (30), dont le signal de sortie est appliqué, conjointement avec le signal électrique pour la sensibilité de déclenchement, à un second amplificateur (40), dont le signal de sortie est appliqué à une entrée d'un premier amplificateur différentiel (60), à l'autre entrée duquel est appliqué le signal électrique qui correspond à la pression dans la conduite d'expiration.

**9.** Appareil respiratoire suivant la revendication 8, caractérisé par le fait qu'entre le second amplificateur (40) et le premier amplificateur différentiel (60) est branché un second amplificateur différentiel (50), à l'autre entrée duquel est appliqué le signal électrique qui correspond au PEEP.

**10.** Appareil respiratoire suivant la revendication 8 ou 9, caractérisé par le fait que le facteur d'amplification k du premier amplificateur (30) est réglable.

FIG 1

## FIG 2

$P[mbar]$

A      B      C

PEEP

2 mbar

$T_{(\Phi)}$

$\Phi_{EXSP} = 0$

$\Phi_{INSP} = \Phi_{EXSP}$     $\Phi_{INSP} > \Phi_{EXSP}$

$\Phi_{INSP} < \Phi_{EXSP}$